(19) Europäisches Patentamt
European Patent Office
Office européen des brevets

(11) **EP 4 118 430 B1**

(12) **EUROPEAN PATENT SPECIFICATION**

(45) Date of publication and mention
of the grant of the patent:
**18.12.2024 Bulletin 2024/51**

(51) International Patent Classification (IPC):
*G01N 33/50* (2006.01)   *G01N 33/483* (2006.01)
*G01N 33/487* (2006.01)   *G01N 33/574* (2006.01)

(21) Application number: **21709435.8**

(52) Cooperative Patent Classification (CPC):
**G01N 33/574; G01N 33/48735; G01N 33/5011;**
**G01N 33/502;** G01N 2800/52

(22) Date of filing: **09.03.2021**

(86) International application number:
**PCT/EP2021/055858**

(87) International publication number:
**WO 2021/180685 (16.09.2021 Gazette 2021/37)**

(54) **MEASURING THE ELECTRIC ACTIVITY OF CELLS FOR THE EVALUATION OF METASTATIC POTENTIAL OF CANCER CELLS**

MESSUNG DER ELEKTRISCHEN AKTIVITÄT VON ZELLEN ZUR BEWERTUNG DES METASTATISCHEN POTENZIALS VON KREBSZELLEN

MESURE DE L'ACTIVITÉ ÉLECTRIQUE DES CELLULES POUR L'ÉVALUATION DU POTENTIEL MÉTASTATIQUE DES CELLULES CANCERES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.03.2020 EP 20161801**

(43) Date of publication of application:
**18.01.2023 Bulletin 2023/03**

(73) Proprietor: **Celex Oncology Innovations Limited**
**London W1W 6XH (GB)**

(72) Inventor: **DJAMGOZ, Mustafa, Bilgin, Ali**
**London SW6 2YF (GB)**

(74) Representative: **Inspicos P/S**
**Agern Allé 24**
**2970 Hørsholm (DK)**

(56) References cited:
• **GUOFENG QIAO ET AL: "Bioimpedance Analysis for the Characterization of Breast Cancer Cells in Suspension", IEEE TRANSACTIONS ON BIOMEDICAL ENGINEERING, IEEE SERVICE CENTER, PISCATAWAY, NJ, USA, vol. 59, no. 8, 1 August 2012 (2012-08-01), pages 2321 - 2329, XP011490168, ISSN: 0018-9294, DOI: 10.1109/TBME.2012.2202904**
• **DIETER HAEMMERICH ET AL: "In vivo electrical conductivity of hepatic tumours; In vivo electrical conductivity of hepatic tumours", PHYSIOLOGICAL MEASUREMENT, INSTITUTE OF PHYSICS PUBLISHING, BRISTOL, GB, vol. 24, no. 2, 1 May 2003 (2003-05-01), pages 251 - 260, XP020073676, ISSN: 0967-3334, DOI: 10.1088/0967-3334/24/2/302**
• **ZURBUCHEN URTE ET AL: "Determination of the temperature-dependent electric conductivity of liver tissue ex vivo and in vivo: Importance for therapy planning for the radiofrequency ablation of liver tumours", INTERNATIONAL JOURNAL OF HYPERTHERMIA, vol. 26, no. 1, 1 January 2010 (2010-01-01), GB, pages 26 - 33, XP055799721, ISSN: 0265-6736, Retrieved from the Internet <URL:https://www.tandfonline.com/doi/pdf/10.3 109/02656730903436442?needAccess=true> DOI: 10.3109/02656730903436442**

- PRAKASH S ET AL: "electrical impedance measurements on excised hepatic tissue from human patients with metastatic colorectal cancer", PHYSIOLOGICAL MEASUREMENT, INSTITUTE OF PHYSICS PUBLISHING, BRISTOL, GB, vol. 36, no. 2, 19 January 2015 (2015-01-19), pages 315 - 328, XP020278221, ISSN: 0967-3334, [retrieved on 20150119], DOI: 10.1088/0967-3334/36/2/315
- SHLOMI LAUFER ET AL: "Electrical impedance characterization of normal and cancerous human hepatic tissue", PHYSIOLOGICAL MEASUREMENT, INSTITUTE OF PHYSICS PUBLISHING, BRISTOL, GB, vol. 31, no. 7, 1 July 2010 (2010-07-01), pages 995 - 1009, XP020175871, ISSN: 0967-3334
- ANGUS MADELINE ET AL: "Voltage gated sodium channels in cancer and their potential mechanisms of action", CHANNELS (AUSTIN), vol. 13, no. 1, 1 January 2019 (2019-01-01), US, pages 400 - 409, XP055800114, ISSN: 1933-6950, Retrieved from the Internet <URL:https://www.ncbi.nlm.nih.gov/pmc/articles/PMC6768049/pdf/kchl-13-01-1666455.pdf>DOI: 10.1080/19336950.2019.1666455

**Description**

FIELD OF THE INVENTION

The present invention relates to the field of cancer diagnosis, prognosis and therapy. In particular, the present invention relates to a method of evaluating the metastatic potential of cancer cells, in particular but not limited to breast cancer cells.

BACKGROUND OF THE INVENTION

Cancer is one of the major killers of humans and many problems remain in its clinical management, from primary diagnosis to treatment of advanced disease.

At present many tumour grading systems are used to assess the metastatic potential of malignant neoplasms. Most methods involve the drawback of relying of an examination of dead tissue derived from the tumour - this is the case if the system describe tumour architecture and/or cytology. As an alternative, motility assays relying on visual inspection of live cells has been employed with some success. However, there exists a need for a reliable and reproducible means for assessing tumour aggressiveness, in particular tumour metastatic potential. None of these grading systems provide any information relating to specific targeted therapy of a malignancy.

Breast cancer (BCa) is the most common type of cancer and leading cause of cancer death in women with over 2 million new cases reported in 2018 (Bray et al., 2018). BCa-related deaths often occur when dysregulated tumour signalling occurs compounded by metastasis (Sever and Brugge, 2015). A strongly metastatic and aggressive form of BCa is the triple-negative breast cancer (TNBC), where genes for the oestrogen receptor (ER) and progesterone receptor (PR) are not expressed, and there is no amplification of the human epidermal growth factor receptor 2 (HER2) (Perou et al., 2000; Lee and Djamgoz, 2018). This renders TNBC particularly difficult to treat as the commonly applied hormone therapies rely on targeting at least one of these three receptors (Al-Mahmood et al., 2018).

The MDA-MB-231 cell line is used commonly as a model of TNBC and has a mesenchymal phenotype (Hero et al., 2019). Its underlying intracellular signalling pathways have been studied using both genomic analyses and expression profiles (Perou et al., 2000; Sorlie et al., 2001; Neve et al., 2006). Indeed, these cells exhibit *in vitro* characteristics representative of cancer cell behaviour *in vivo* (Sever and Brugge, 2015).

In recent years, expression of ion channels and transporters (ICTs) has been implicated in various stages of the cancer process, such as cell proliferation, apoptosis, migration, and invasiveness (Lastraioli et al., 2015). This highlights the importance of studying cancer cell electrophysiology. In particular, voltage-gated sodium channels (VGSCs) may play a potentiating role in metastasis (Arcangeli et al., 2008; Fraser and Pardo, 2008; Prevarskaya et al., 2010; Cuddapah and Sontheimer, 2011; Djamgoz et al., 2014; Djamgoz et al., 2019). VGSCs are typically known for their role in producing action potentials in excitable cells, such as neurons and myocytes.

Djamgoz advanced the view ("Celex Hypothesis") that it is the *de novo* VGSC expression and the resulting "membrane excitability" that would promote cancer cell aggressiveness and, ultimately, metastasis (Djamgoz, 2011; Djamgoz et al., 2014). Such studies have been aided by the availability of rich pharmacology for ion channels, as well as genetic studies. In human and rodent cell lines, single-cell patch clamp recordings revealed that VGSCs can be blocked specifically with tetrodotoxin (TTX) (Grimes et al., 1995; Roger et al., 2003). In turn, TTX application would lead to suppression of invasiveness in vitro and metastasis in vivo (Grimes et al., 1995; Roger et al., 2003; Fraser et al., 2005; Yildirim et al., 2012; Djamgoz et al., 2019).

Breast cancer is one of the most prevalent types of cancers worldwide and yet, its pathophysiology is poorly understood. This is particularly the case for the TBNC subtype. Single-cell electrophysiological studies have provided evidence that membrane depolarisation is implicated in the proliferation and metastasis of breast cancer. However, metastatic breast cancer cells are highly dynamic microscopic systems with complexities beyond a single-cell level. There is an urgent need for technologies capable of decoding the intercellular signalling pathways and networks that control proliferation and metastasis, particularly at a population level.

OBJECT OF THE INVENTION

It is an object of embodiments of the invention to provide methods for assessing the metastatic potential of cancer cells, in particular of breast cancer cells.

SUMMARY OF THE INVENTION

Single-cell patch clamp recording is used for investigating membrane currents or voltages associated with ion channels upon the application of an external stimulus, under current or voltage clamp conditions, respectively. Whilst this sort of intracellular monitoring under an applied electrical stimulus can provide detailed information at the cellular level, the technique would suffer from the imposition of a micro-pipette electrode the volume of which is much bigger than the cell. Furthermore, for reliable *in vitro* models and studies of phenomena such as cancer cell invasion, the application of an external stimulus is likely to influence natural interlinked cell-cell dynamics. Additionally, cancer cell proliferation, invasion and metastasis typically involve a cell cohort, and hence it would be advantageous to monitor the network phenomena together with the electrical activity of single cells. Changes in the cell cohort during metastasis, when cancer cells spread to tissues and organs beyond the original tumour's location, are of paramount clinical importance. Hence, patch clamping alone lacks the ability for non-invasive network mapping within the tumour bulk. Monitoring unbiased cell invasion at a population level coupled with individual cellular measurements is therefore critical for understanding tumour proliferation, invasion and metastasis.

It has been found by the present inventor(s) that it is possible by non-invasive *in vitro* means to obtain electrical recordings of strongly metastatic MDA-MB-231 and weakly/non-metastatic MCF-7 breast cancer cell lines and that the recordings correlate with the metastatic activity/potential of the cancer cells.

To accomplish this, use was made of an ultra-low noise sensor that exploits large-area electrodes, of 2 mm$^2$, which maximizes the double-layer capacitance and concomitant detection sensitivity (Rocha et al., 2016a). It was demonstrated that the current recorded after adherence of the cells is dominated by the opening of voltage-gated sodium channels (VGSCs), confirmed by experiments including application of the highly specific inhibitor, tetrodotoxin (TTX). The electrical activity of MDA-MB-231 cells was demonstrated to surpass that of the MCF-7 cells, strongly suggesting a link between the cells' bioelectricity and invasiveness. An activity pattern with characteristics similar to that of Random Telegraph Signal (RTS) noise was also demonstrated. RTS patterns were less frequent than the asynchronous VGSC signals. The RTS noise power spectral density showed a Lorentzian shape, which revealed the presence of a low-frequency signal across MDA-MB-231 cell populations with propagation speeds of the same order as those reported for intercellular $Ca^{2+}$ waves.

The invention thus paves the way for real-time investigations of the bioelectricity of cancer cells, their ionic/pharmacological properties, and their relation to metastatic potential of the cells.

The present invention unambiguously shows that human breast cancer cells are *not* electrically inert as might be expected; extracellular electrical signals *in vitro* are detected in a population of MDA-MB-231 cells, by means of the ultrasensitive measurement setup based on low-impedance electrodes. The electrical behaviour of MDA-MB-231 cells reveals an asynchronous pattern identified primarily as the product of their VGSC activity, as demonstrated by TTX inhibition experiments. In particular, it is demonstrated that strongly metastatic MDA-MB-231 cells are electrically active with VGSC-dependent spontaneous spiking, whilst the MCF-7 cells (the control), showed only very low-level electrical excitability.

This strongly suggests a link between electrical excitability and metastasis. In addition, a slower signal pattern with similar characteristics to that of RTS noise was detected. The calculated wave speed, based on the RTS pattern, was in agreement with previously reported values for intercellular $Ca^{2+}$ waves.

It is believed that these findings will open new avenues for more accurate pharmacological investigations and action of drugs on cancer cell ensembles, in a personalised clinical setting.

So, in a first aspect the present invention relates to a method for determining the metastatic and/or invasive potential of a malignant neoplasm, the method comprising recording electrical activity in a sample of cells isolated from the malignant neoplasm and comparing recorded electrical activity of the sample with electrical activity of one or more reference cell samples having a defined metastatic and/or invasive potential and subsequently assigning a score for the metastatic and/or invasive potential of the malignant neoplasm by ranking the recorded electrical activity relative to the electrical activity of the one or more reference samples, wherein the reference cell samples preferably are at least a cell sample from a highly metastasizing cancer and a cell sample from a non-metastatic or low-metastasizing cancer, preferably a cancer of the same tissue origin and histological origin,
wherein said electrical activity is constituted by variations in the membrane potential relative to the resting potential measured in the sample of cells.

In a second aspect, the invention relates to a method for classifying the therapeutic potential of an agent or a combination of agents for prevention and/or inhibition of metastasis and/or invasive growth by a malignant neoplasm in a patient, the method comprising determining the metastatic and/or invasive potential of the malignant neoplasm according to the method of the first aspect of the invention and under conditions 1) in the absence of the agent or combination of agents and 2) where the agent or combination of agents is or has been added, wherein the agent or combination of agents is classified as having a therapeutic potential for treatment of the patient if it decreases the assigned score for metastatic and/or invasive potential in an in vivo acceptable concentration.

LEGENDS TO THE FIGURE

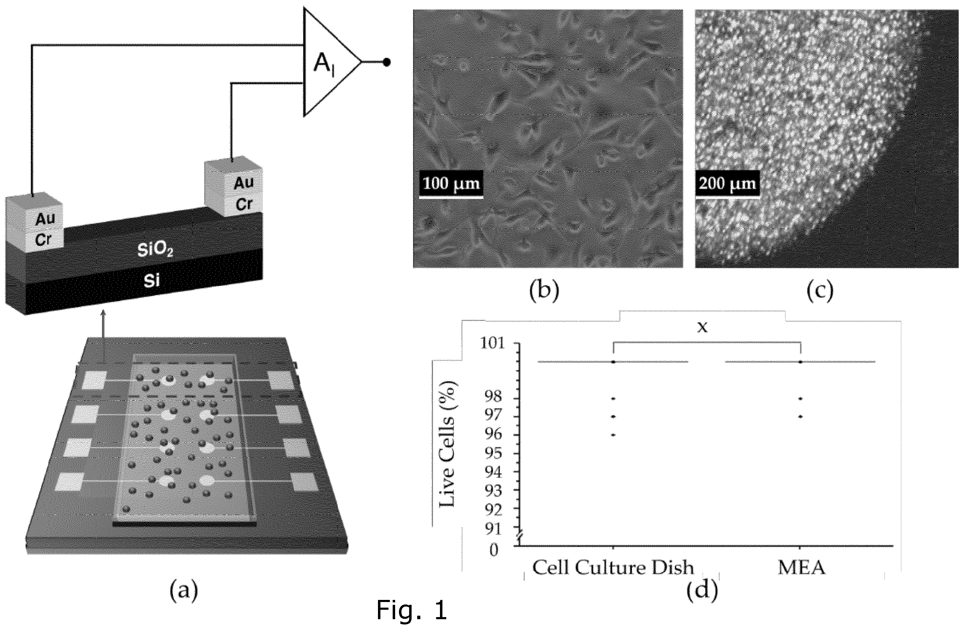

Fig. 1: MEA device useful in the method of the invention

(a) Representative diagram of the MEA device with a layer of adherent cells. The layers of materials used and connections to the low-noise current amplifier (A) are also shown above the MEA. A cell density of $1 \times 10^6$ cells/ml was deposited, forming a confluent layer across all the electrodes.
(b) Morphology of MDA-MB-231 cell cohort as seen under a light
(c) Confluent layer of MDA-MB-231 cells adhered to one of the 2 mm$^2$ electrodes. The dark area on the right shows the substrate and the light circular area to the left shows the electrode.
(d) Box plot from cell viability studies indicating no significant statistical difference between viability of cells on the MEA versus culture dish ("X" represents P = 0.98; n = 4 for both).

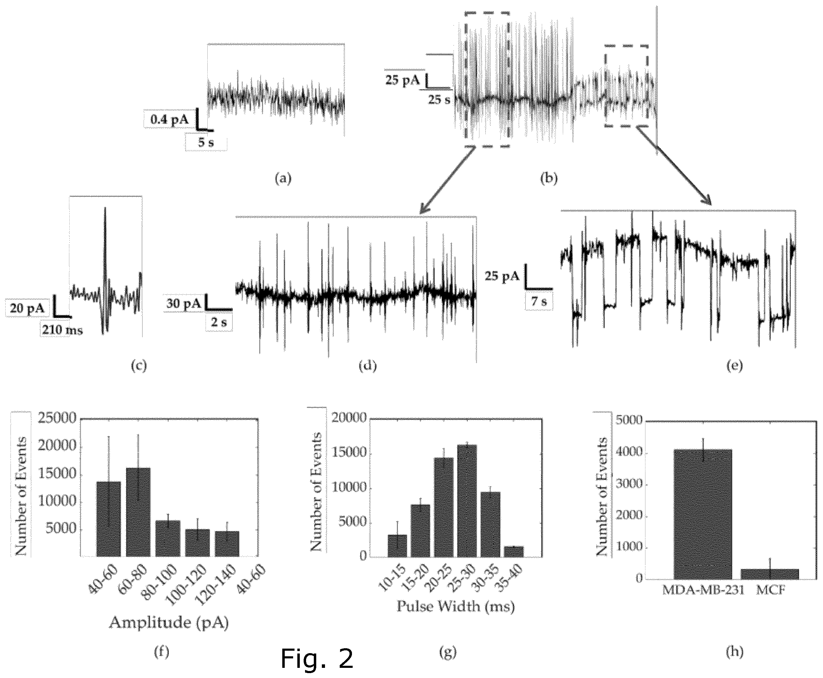

Fig. 2: Electrical activity of MDA-MB-231 cells recorded over a period of up to three days.

(a) Baseline current measured with only medium over the electrodes' surface, showing a current of 0.5 pA. (b) Typical asynchronous spiking activity. (c) An expanded trace showing a fast, asynchronous spike. (d) Detailed section of fast spiking activity. (e) Detailed section of square shaped pulses.
(f - g) Results from spike characterization over a 12-hour period of asynchronous spiking activity of MDA-MB-231 cells, showing the distribution of amplitudes with bin intervals of 20 pA each (f) and pulse widths with bin intervals of 5 ms each (g) Bars represent average values of all 3 repeats and error bars represent SDs.
(h) Experiment results comparing the spike counts obtained from 24-hour measurements on strongly metastatic MDA-MB-231 cells versus weakly metastatic MCF-7 cells, showing a significantly smaller spike count for the latter.

Fig. 3: VGSC activity of MDA-MB-231 cells. VGSC 406 activity was blocked using TTX (20 μM).

(a) Current trace showing electrical activity before, during and after application of TTX.
(b) Quantification of the spikes recorded in (a). Number of spikes were measured in time bins of 6 minutes. Bars represent average values of all 3 repeats and error bars represent SDs.

Fig. 4: Graphs showing Random Telegraph Signal (RTS) noise.

(a) Characterisation of RTS "up" state.
(b) Characterisation of RTS "down" state.
(c) PSD for the RTS noise, showing a Lorentzian spectrum.

DETAILED DISCLOSURE OF THE INVENTION

*Definitions*

The expression "metastatic potential" refers to the ability of a malignant neoplasm to metastasize outside its current location in the body. The quantification of metastatic potential can take any convenient form. For instance, malignant neoplasms can be scored as "non-metastatic", "low-metastatic", and "high-metastatic" (or even more simple: "low-metastatic" and "high-metastatic") based on clinical experience. Alternatively, numerical scores may be used based on any of several clinical observations (rate of formation of metastases, anatomical spread of new metastases, growth rate of metastases etc.). In the present invention, any of such grading systems can be utilised, because the neoplasm to be examined ultimately is compared to one or more cancers that have a previously established metastatic behaviour classified by any of these systems.
Likewise, the expression "invasive potential" refers to the ability of a malignant neoplasm to invade neighbouring tissue. The quantification of invasive potential can take any convenient form. For instance, malignant neoplasms can be scored as "non-invasive", "low-invasive", and "highly invasive" (or even more simple: "low-invasive" and "high-invasive") based on clinical experience. Alternatively, numerical scores may be used based on any of a number of clinical observations (rate of formation of metastases, anatomical spread of new metastases, growth rate of metastases etc.). In the present invention, any of such grading systems can be utilised, because the neoplasm to be examined ultimately is compared to one or more cancers that have a previously established invasiveness classified by any of these systems.
"Electrical activity" of cells in the present context refers to recordable variations in the membrane potential (relative to the resting potential) measured in a population of cells; in turn, such variations in the membrane potential gives rise to measurable electrical currents. This can be compared to the electrical activity found in excitable cells such as neurons and myocytes, but as demonstrated herein the frequency of the membrane potential variations is much lower than the normally observed kHz range variations recorded from excitable cells. The electrical activity is typically termed "spikes" due to the appearance of variations when studying the membrane potential or electric current in the time domain.

The term "malignant neoplasm" (also termed "cancer", and, if solid, "malignant tumour") relates to a population of cells that exhibit the hallmarks of malignant cells: i.e. uncontrolled and uncoordinated growth, the ability to traverse tissue barriers (i.e. invasiveness), immortality or at least reduced programmed cell death, the ability to promote neovascularization, and metastatic growth.

A "reference cell sample" is a collection of cells, typically in the form of clonal cells or a cell line, which has a known capacity for invasive grown and metastatic behaviour. Examples of reference cells useful in the present invention are the breast cancer derived cell lines MDA-MB-231 (highly metastatic) and MCF-7 (low metastatic), but human cell lines of varying metastatic/invasive capacity are known from a large range of neoplasms. Such cell lines are commercially available and can e.g. be acquired from Sigma Aldrich (www.sigmaaldrich.com/life-science/cell-culture/cancer-cell-lines.html) or ATCC. Human cell lines particularly useful as reference are: PC-3/M, LNCaP (prostate cancer); SW620, SW48, HT29, HCT116 (colorectal cancer); H460, A549, H69, H209, H510 (lung cancer); SKOV-3, Caov-3 (ovarian cancer); HeLa, C33A, SiHa, CaSki (cervical cancer); BGC-823 and MKN-28 cells (stomach cancer); PANC-1, Mia PaCa-2 and BxPc3 (pancreatic cancer).

A voltage-gated sodium channel (VGSC) is an integral membrane protein forming a selective sodium ion channel through the cellular plasma membrane, which exhibit 3 main conformational states (closed, open, and inactive). The closed channel becomes permeable (open) for $Na^+$ ions at a certain increase in the cell's membrane potential, causing a rapid further increase in the membrane potential until a threshold level is reached at which the channel is inactivated leading to a gradual reversion to the resting potential. During this reversion, the channel shifts from the inactivated state to the closed state where it can again be opened by an increase in membrane potential. VGSC are found in excitable cells, but have also been demonstrated in malignant cells, where they have been associated with the characteristics of invasiveness and metastatic behaviour.

A "VGSC inhibiting agent" (also termed a "VGSC blocker") is a substance that inhibits or completely blocks the $Na^+$ transport by a VGSC. For the purpose of testing the VGSC contribution to electrical activity exhibited by a population of cancer cells, a convenient choice for a VGSC blocker is the highly toxic substance tetrodotoxin (TTX). For therapeutic purposes including each and any therapeutic aspect and embodiment disclosed herein, a number of other substances are useful: these include without limitation Ranolazine (N-(2,6-Dimethylphenyl)-4-[2-hydroxy-3-(2-methoxyphenoxy)propyl]-1-piperazineacetamide), Eleclazine (4-(pyrimidin-2-ylmethyl)-7-(4-(trifluoromethoxy)phenyl)-3,4-dihydrobenzo[f][1,4]oxazepin-5(2H)-one), 4-(pyrimidin-2-ylmethyl)-7-(4-(trifluoromethyl)phenyl)-3,4-dihydrobenzo[f][1,4]oxazepin-5(2H)-one, and Riluzole (6-(Trifluoromethoxy)-2-benzothiazolamine).

*Specific embodiments of the invention*

In general, the disclosure below relates primarily to methods that target human malignant neoplastic diseases, so preferred embodiments of all aspects of the invention relate to human malignant neoplasms and human patients, wherever applicable. However, treatment of other animals, primarily mammals, is not excluded.

First aspect of the invention

As indicated above, the first aspect relates to a method for determining the metastatic and/or invasive potential of a malignant neoplasm, the method comprising recording electrical activity in a sample of cells isolated from the malignant neoplasm and comparing recorded electrical activity of the sample with electrical activity of one or more reference cell samples having a defined metastatic and/or invasive potential and subsequently assigning a score for the metastatic and/or invasive potential of the malignant neoplasm by ranking the recorded electrical activity relative to the electrical activity of the one or more reference samples, wherein the reference cell samples preferably are at least a cell sample from a highly metastasizing cancer and a cell sample from a non-metastatic or low-metastasizing cancer, preferably a cancer of the same tissue origin and histological origin,
wherein said electrical activity is constituted by variations in the membrane potential relative to the resting potential measured in the sample of cells.

Ranking of the recorded electrical activity and assigning the score will typically entail that a standard 2D Cartesian coordinate system is established based on one type of electrical activity measurement values from relevant reference cell samples and a measure for their metastatic and/or invasive potential. Interpolation between data points for each reference sample will allow that a measured value of electric activity can be translated into a measure of metastatic and/or invasive potential. In other words, the recorded electrical activity is compared to electrical activities from each of a plurality of reference cell samples in order to allow a determination of the metastatic and/or invasive potential of the malignant neoplasm being tested. The electrical activity used in the analysis can for instance be one or more of amplitude, duration/width, frequency, number, shape, or pattern of spikes of electric current or membrane potential. For instance, as shown herein malignant metastasizing cells exhibit an RTS noise power spectral density having a Lorentzian pattern, meaning that inclusion of a measurement of RTS noise appears and its spectral density would be relevant.

It is also possible to include more complex data sets from the reference samples, i.e. more than one of the above listed possible electrical activities. In such a case the standard curve can be established e.g. via multivariable regression analysis (*i.e.* multivariate regression analysis, with only one dependent variable and several independent variables - in this case the metastatic and/or invasive potential is the dependent variable and various measures of electrical activity are the independent variables). In order to deduce the metastatic potential, the measured electrical values are then applied to the formula for the regression analysis to arrive at the score value.

In its simplest form, only one reference sample is used - typically from a highly or moderately metastasizing neoplasm. Since a cell having zero metastatic potential will have a negligible (zero) electrical activity, the data point corresponding to the origin of the axes in the coordinate system will constitute an artificial reference sample point for a reference sample from a cell population with no metastatic and/or invasive potential. However the reference cell samples are normally at least a cell sample from a highly metastasizing cancer and a cell sample from a non-metastatic or low-metastasizing cancer, preferably a cancer of the same tissue origin and histological origin; the latter ensures that the correlation between electric activity and metastatic/invasive potential is based on cells that otherwise are comparable, thus minimizing the risk that inclusion of cells of different origins could provide unreliable results.

In particular important embodiments, the malignant neoplasm is a breast cancer and, in that case, useful and important reference cell samples include or consist of MDA-MB-231 and MCF-7 cells.

However, any malignant neoplasm - typically a solid tumour - could constitute the neoplasm subjected to the method of the first aspect of the invention. Hence other malignant neoplasms are selected from the group consisting of an epithelial tumour, a non-epithelial tumour, and a mixed tumour. The epithelial tumour may be both a carcinoma or an adenocarcinoma, and the non-epithelial tumour or mixed tumour is typically a liposarcoma, a fibrosarcoma, a chondrosarcoma, an osteosarcoma, a leiomyosarcoma, a rhabomyosarcoma, a glioma, a neuroblastoma, a medullablastoma, a malignant melanoma, a malignant meningioma, a neurofibrosarcoma, a leukemia, a myeloproleferative disorder, a lymphoma, a hemangiosarcoma, a Kaposi's sarcoma, a malignant teratoma, a dysgerminoma, a seminoma, or a choriosarcoma. Also, the anatomic location of the malignant neoplasm can be anywhere in body. So malignant neoplasm may be a of the eye, the nose, the mouth, the tongue, the pharynx, the oesophagus, the stomach, the colon, the rectum, the bladder, the ureter, the urethra, the kidney, the liver, the pancreas, the thyroid gland, the adrenal gland, the breast, the skin, the central nervous system, the peripheral nervous system, the meninges, the vascular system, the testes, the ovaries, the uterus, the uterine cervix, the spleen, bone, or of cartilage.

The method of the first aspect typically entails that the electrical activity is measured by recording electric currents and/or potentials with a multi-electrode array capable of measuring low-frequency electrical events in the sample, such as events with a frequency <1 kHz.

The technical challenge involved in measuring electric activity of cancer cells is that the membrane currents associated with VGSC activity in cancer cells possess amplitudes which are orders of magnitude smaller compared to those observed in neurons. Commercially available electrophysiology systems such as multi-electrode arrays (MEAs) comprise a high-density array of electrodes that are either planar or three-dimensional, with a diameter ranging between 10-100 $\mu$m typically embedded on top of an insulating MEA substrate (Spira and Hai, 2013). This set-up allows for neuronal cells to adhere and their electrical activity readily recorded and modulated.

However, the size of the electrodes from such devices contributes towards debilitatingly high impedances and the monitoring window is set in the kHz range where neuronal firing typically occurs. Low-frequency events are therefore filtered out. According to the present invention and to overcome these drawbacks and technical challenges, a sensitive detection method was utilised based on large electrodes, with areas in the order of mm$^2$. These low-impedance electrodes make use of a large Helmholtz-Gouy-Chapman double-layer capacitance. The double layer acts as a multiplication factor to the displacement current produced by cells adhered on the electrode surface, allowing for small-amplitude signals to be detected (Rocha et al., 2016a; Rocha et al., 2016b; Cabello et al., 2019).

Herein is demonstrated that the strongly metastatic BCa cells can be electrically monitored using this sensitive electrode system. The recordings from MDA-MB-231 cells revealed the presence of asynchronous spikes with magnitudes ranging from 40 pA to 140 pA. The electrical activity of MDA-MB-231 cells significantly surpasses the attenuated electrical activity of the weakly metastatic cell line, MCF-7. By using the highly specific pharmacological inhibitor TTX it was demonstrated that the asynchronous spikes are primarily caused by the opening and closing of the VGSCs, in direct agreement with VGSC expression occurring specifically in BCa cells of strong metastatic potential (Fraser et al., 2005). In addition, sporadic occurrence of "Random Telegraph Signal" (RTS) noise was noted. RTS patterns were less frequent than the asynchronous VGSC signals. Characterisation of the RTS noise revealed the presence of a low-frequency signal across the cell cohort with propagation speeds of the same order as those reported for intercellular "Ca$^{2+}$ waves" (Rizaner et al., 2016). These findings suggest that the electrical activity recorded from the MDA-MB-231 cells is a metastatic phenomenon. The recording system enables the real time investigation of the bioelectricity of BCa cells and corroborant cohort signalling.

Therefore, in all embodiments disclosed herein where the electrical activity of a sample of cells is recorded, it is preferred that a low-impedance MEA device is used and the teachings from Rocha et al., 2016a, Rocha et al., 2016b, and Cabello et al., 2019 in relation to the measurement device can be applied to the present invention. Hence the electrodes of the multi-electrode array each have preferred contact areas >1 mm$^2$, such as about 2 mm$^2$.

Preferably the electrodes are covered by or made from an inert material such as gold, platinum, and rhodium, thus providing for an "inert electrode" which is not electrochemically active but merely transport electric charge.

During the recording of the electric activity, it is preferred that the cells of the sample adhere to the electrode. To achieve this goal, the electrode is typically treated with a substance that facilitates cell adherence. Such substances are known in the art - examples are poly-L-lysine, fibronectin, laminin, and collagen. Regardless of the means used for this purpose, it is preferred that the cells of the sample form a confluent layer across the electrodes during recording.

As shown in the examples, the predominant electrical activity observed in a highly metastatic cell line was constituted by current/membrane potential spikes from currents in VGSC's. Since previous research has revealed that metastatic behaviour at least in part can be attributed to expression of VGSC by cancer cells, it is therefore of interest to verify that a broad population of cells from a malignant neoplasm, which according to the method of the first aspect of the invention has a high metastatic potential because they express active VGSCs - this would warrant that the patient is treated with pharmaceutical agents that block VGSCs in order to prevent further metastatic and invasive growth. To achieve this goal, the method of the first aspect can therefore include that multiple recordings are made, wherein some recordings are made in the presence of a VGSC (voltage gated sodium channel) inhibiting agent, such as tetrodotoxin (which is a very effective blocker of VGSCs). In this embodiment, the electrical activity is typically recorded under a plurality of concentrations of the VGSC inhibiting agent and/or the electrical activity is recorded in a period after addition of the VGSC inhibiting agent and/or in a period after the concentration of the VGSC inhibiting agent has been reduced.

Second aspect of the invention

This aspect relates to method for classifying the therapeutic potential of an agent or a combination of agents for prevention and/or inhibition of metastasis and/or invasive growth by a malignant neoplasm in a patient, the method comprising determining the metastatic and/or invasive potential of the malignant neoplasm according to the method of the first aspect of the invention and any embodiment thereof 1) in the absence of the agent or combination of agents and 2) where the agent or combination of agents is or has been added, wherein the agent or combination of agents is classified as having a therapeutic potential for treatment of the patient if it decreases the assigned score for metastatic and/or invasive potential in an in vivo acceptable concentration.

Put in simpler terms, this aspect relates to identification of a drug or drug combination, which is effective in treating the individual patient based on the metastatic/invasive potential of the patient's cancer AND on the efficacy of the tested drug in reducing the electric activity of the cancer cells and thereby reduce the metastatic/invasive potential of the cancer. Typical effective drugs in that respect are the VGSC blockers described herein as effective pharmaceuticals, cf. above, but any drug or drug combination that passes the test of being able to reduce the electric activity will be considered useful in treatment.

Combination drugs are preferably at least one VGSC blocker and a potassium channel opener, cf. the disclosure in European patent application number 19205075.5. Suitable potassium channel openers in such a combination with a VGSC blocker are selected from the group consisting of Minoxidil (6-(1-Piperidinyl)pyrimidine-2,4-diamine 3-oxide), Amiloride (3,5-diamino-6-chloro-N-(diaminomethylidene)pyrazine-2-carboxamide), AG1478 (N-(3-chlorophenyl)-6,7-dimethoxy-4-quinazolinamine), Aprikalim, Bimakalim, Cromakalim,, Diazoxide, Emakalim, Levcromakalim, Mazokalim, Naminidil, Nicorandil, Pinacidil, Pilmakalim, Sarakalim, Flufenamic acid, Meclofenamic acid, Niflumic acid, Nimesulide, Rottlerin (mallotoxin), Tolfenamic acid, Lupirtine, Retigabine, and Riluzole.

The method of the 2nd aspect is in a preferred embodiment carried out under hypoxic conditions, cf. the disclosure in WO 2012/049440, which discloses that VGSC blockers are more effective in reducing surrogate measures of metastatic/invasive behaviour under hypoxia.

Therapies enabled by the invention

The 2nd aspect of the inventions enables a method for treatment of a patient, such as a human patient, of a malignant neoplasm, the method comprising obtaining a sample of cells from the malignant neoplasm, classifying at least one agent or combination of agents vis-à-vis the sample according to the of the 2nd aspect of the invention and any embodiment thereof, and subsequently administering to the patient at least one of said agent or combination of agents that are classified as having a therapeutic potential by the method of the 2nd aspect. Consequently, the method relates to personalized medicine, where the patient's tumour is "typed" relative to its metastatic/invasive potential, and where drug(s) is/are identified that will reduce the metastatic/invasive potential as evidenced by its/their ability to reduce the electric activity - this/these drugs are then used in the subsequent treatment of the patient.

The treatment can be combined with treatment with another anti-cancer drug, such as a cytostatic drug, a hormone blocker or checkpoint inhibitor. Such drugs could include Gefitinib, Tamoxifen, Letrozole, Flutamide, Nivolumab, Pembrolizumab.

In the event the at least one agent is or comprises a VGSC blocker, the treatment can in preferred embodiments entail combination with a potassium channel opener, e.g. one of those potassium channel openers listed above.

Also enabled is a method for treatment of a patient, such as a human patient, of a malignant neoplasm, the method comprising administering an effective (and pharmaceutically acceptable) amount of an agent that reduces VGSC current, in particular the transient VGSC current, to the patient, wherein the effective amount is non-lethal to the cells of the malignant neoplasm and reduces or blocks invasive and/or metastatic growth of the cells of the malignant neoplasm, and wherein the metastatic and/or invasive potential of the cells of the patient's malignant neoplasm has been determined according to the method of the first aspect of the invention as described above under the Summary of the Invention. In addition, the agent that reduces VGSC current can also be tested in the method of the $2^{nd}$ aspect and its embodiments in order to verify that it will indeed reduce the metastatic/invasive potential of the malignant neoplasm.

The rationale is that the demonstration that the electrical activity can be reduced by applying a VGSC blocker in the method of the $1^{st}$ aspect demonstrates that the malignant cells are sensitive to VGSC blocking in general. By also adding on the method of the $2^{nd}$ aspect, a further verification is provided.

The agent administered can be any of the above-mentioned VGSC blockers that are pharmaceutically effective. The effective amount is in preferably non-lethal to the cells of the malignant neoplasm while reducing or blocking invasive and/or metastatic growth of the cells of the malignant neoplasm, see also WO 2012/049440.

Also, combination treatment with any one of the potassium channel openers disclosed above is possible .

EXAMPLE 1

*Electrophysiology of population of cancer cells in vitro*

Materials and Methods

**Maintenance of breast cancer cell lines**

The MDA-MB-231 and MCF-7 cells were cultured in Dulbecco's Modified Eagle Medium (DMEM; Sigma) containing 5% v/v foetal bovine serum (FBS; Gibco) and 4 mM L-Glutamine (Sigma). The cells were harvested once a confluence of 70% or over was achieved. The cells were washed with phosphate buffered saline (PBS; Sigma) and dissociated using 0.05% Trypsin-EDTA solution (Sigma) at 37 °C for 3 minutes. A cell density of $1\times106$ cells/ml was deposited on the device for each experiment. Images were acquired using either an M205 Leica stereo microscope (cells on electrodes) or a Leica DMIRB microscope fitted with a Nikon DXM1200C digital camera.

The trypan blue exclusion assay was used to determine viability of cells plated on MEAs compared to the tissue culture dish (Fraser et al., 2005). Briefly, a MEA was placed in a 35 mm tissue culture dish (Falcon) and pre-treated for 20 min with 0.01 mg/ml poly-L-lysine (Sigma) to promote cell adhesion. Cells were plated at a density of $1\times10^6$ cells/ml and allowed to attach overnight. The media was then replaced with 0.1% trypan blue solution. Following incubation for 10 min at 37°C, the trypan blue solution was replaced with 1 ml fresh cell culture medium and the cells were viewed at 100x magnification under a dissection microscope (Vickers Instruments). The percentage of dead cells was determined from 30 randomly selected fields of view for either the MEA or the dish. This procedure was repeated in 4 independent experiments.

**MEA design and manufacturing**

The MEA device consisted of a silicon dioxide substrate onto which four pairs of circular electrodes were deposited, each with an area of 2 $mm^2$. This was achieved by evaporating a 10 nm layer of chromium followed by a 50 nm layer of gold through a shadow mask with the desired electrode design. The electrodes were connected to measurement pads at the edges of the device through strip lines of negligible area. In order to contain the culture medium and cells over the electrodes, a previously manufactured Poly(methyl methacrylate) (PMMA) well was secured onto the MEA before plating the cells.

The MEA was then further enclosed in an autoclaved container and the electrodes covered in 0.01 mg/ml poly-L-lysine (Sigma) to promote cell adhesion. This was applied and left for 5 minutes, washed three times with MilliQ water and left to stand for 1.5 hours prior to depositing the cells. Measurements started at least one hour after the cell seeding.

The container with the seeded MEA was kept in an incubator (Midi 40; Thermo Scientific) at 37°C and 5% $CO_2$ and connected to low-noise cables to perform the electrical measurements. The current between the 2 $mm^2$ electrode pair was measured using a low-noise current amplifier (SR570; Stanford Research) with an amplification factor of 10 nA/V. This data was then plotted using a dynamic signal analyser (35670A; Agilent) and LabVIEW software connected via a high speed GPIB interface. The instrumentation was further contained within a Faraday cage and connected with low-noise cables to minimise external interference.

**Pharmacology**

To determine the origin of the electrical activity and following prior research relating to the role of VGSCs in metastasis (Fraser et al., 2005; Chioni et al., 2009), a highly specific VGSC inhibitor, TTX (Alomone Labs), was applied to the cells in culture. Three experiments were conducted using 20 $\mu$M, following the reported value of TTX toxicity in earlier studies (Fraser et al., 2005). For these experiments, 2 $\mu$l of 10 mM TTX was combined with 20 $\mu$l of the culture medium on the MEA and the mixture reintroduced into the system, obtain final concentrations of 20 $\mu$M of TTX in 1 ml volume on the chip. This was left for an hour and the recording of the electrical current of the cell cohort was continued. Finally, the MEA was washed thrice with fresh medium at 37°C. The recovery of the ensuing electrical behaviour was seen shortly after washing the TTX out and monitored over an additional 24 hours.

Results and Discussion

**Detection method and sensor viability**

Fig. 1(a) shows the design and materials chosen for the MEA devices which were used to perform the extracellular measurements. The sensor comprises two circular electrodes, with one of the electrodes acting as a measuring electrode and the other serving as a counter electrode. The interface between the cells and the electrodes can be described using an equivalent circuit model. When an electrolyte is in close contact with an electrode, a Helmholtz-Gouy-Chapman double layer is formed consisting of a resistance, $R_D$ in parallel with a capacitance, 165 $C_D$. Cells deposited on the measuring electrode, generate a voltage, $v_s(t)$. The resultant signal loss is modelled by a spreading resistance, $R_C$. A transimpedance amplifier was used in this setup to measure the corresponding displacement current, $i_s(t)$, as described earlier (Medeiros et al., 2016):

$$iS(t) = dvS/dt \cdot C_D(1 - e^{(-1/\tau)}) \qquad (1)$$

where $T \cong R_C C_D$ and corresponds to the time constant for the charging and discharging of the network. $C_D$ acts as a multiplication factor and is dependent on the electrode area. The spatial resolution is considerably reduced due to the large electrode area. The measured current signal is the sum of each individual cell's contribution on the electrode surface. Under the culture conditions used, the MDA-MB-231 cells maintained their familiar mesenchymal phenotype (see Fig. 1(b)) and adhered well to the electrodes (see Fig. 1(c)). Furthermore, their viability was not affected by contact with the electrode (see Fig. 1(d)).

**Recording of asynchronous electrical currents**

The bioelectricity of the MDA-MB-231 cell populations was recorded over time, for up to 72 hours. All electrical recordings were repeated in three independent experiments. Prior to introducing the cells, the baseline current seen using only cell culture medium on gold (Au) electrodes coated with Poly-L-Lysine was about 0.5 pA, as shown in Fig. 2(a) and in agreement with previous work (Rocha et al., 2016b).

Fig. 2(b) shows the two typical spiking patterns recorded from MDA-MB-231 cells. The first electrical pattern consists of asynchronous bipolar spikes. A high magnification asynchronous spike is depicted in Fig. 2(c) and a group of these bipolar spikes is shown in Fig. 2(d). The average spike magnitude ranged from 40 to 140 pA, most events falling between 60 to 80 pA, as seen in Fig. 2(f). To further quantify the recorded asynchronous pattern, the data from three different experiments was analysed and the distribution of asynchronous spike widths was extracted. The distribution follows a Gaussian curve with a peak at around 30 ms, in good agreement with the timescales reported using patch clamp recordings on MDA-MB-231 cells (Fraser et al., 2005). The rare presence of spike magnitudes reaching up to 300 pA with spike widths of a few seconds was noted. These were less frequent as depicted in the event count of Fig. 2(f). As a control, the electrical activity of MCF-7 cells was also measured in the same conditions as the MDA-MB-231 cells. MCF-7 cells are known to be weakly/non-metastatic and do not express functional VGSCs (Fraser et al., 2005; Rizaner *et al.,* 2016) and hence should exhibit reduced electrical activity in comparison to MDA-MB-231 cells. The electrical activity for both cell lines was compared as a spike count over the course of 24 hours and is shown in Fig. 2(h). The spike count comparison, over three different experiments, confirms that MDA-MB-231 cells are 90% more electrically active than the MCF-7 cells. The MCF-7 cells sporadically exhibited bipolar spikes. These results suggested that electrogenicity of cancer was associated with cells of strong metastatic potential.

**Effect of TTX**

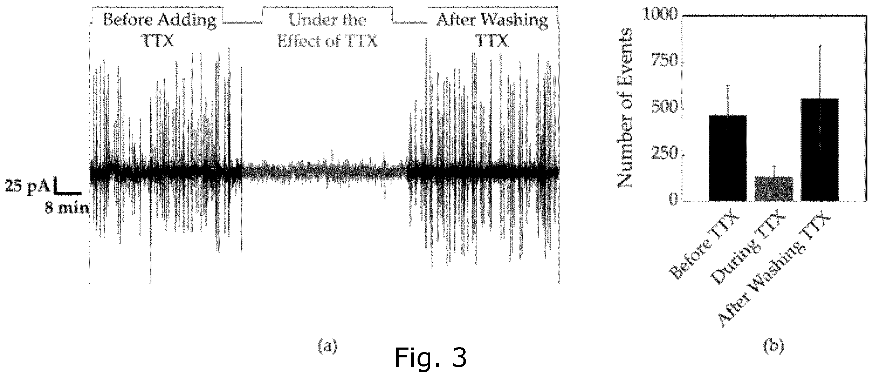

In order to demonstrate the dependence of electrical activity on the presence of functional VGSCs, cells were treated with the neurotoxin TTX (Fig. 3). Under normal culture conditions, cells were electrically active exhibiting asynchronous spikes with amplitudes of 60 - 150 pA (Fig. 3).

Following the addition of TTX, this electrical activity decreased, with currents reaching up to 20 pA (Fig. 3(a)). Upon the removal of TTX, the electrical activity of the MDA-MB-231 cells resumed, as highlighted and quantified with spike counts (Figs. 3(a) and (b)). The time bins in Fig. 3(b) correspond to six minutes portions before, during, and after TTX. Before the TTX was added, an average of $460\pm180$ spikes was observed. This count reduced to an average of $130\pm60$ after 1-2 minutes while TTX was present. After the TTX was washed out, the number of spikes increased to $550\pm280$, and the original asynchronous spikes returned. These results demonstrate a link between the fast, electrical "spiking" of the MDA-MB-231 cells and VGSC activity. TTX, a well-known VGSC blocker, significantly reduced the extracellular electrical activity. Upon washout of the TTX, the cells resumed their previous electrical activity, confirming the rapid reversibility of the TTX action and the lack of any toxicity from the TTX concentration used.

**Random Telegraph Signal noise**

RTS noise can be characterised in terms of its "up" and "down" time. The "up" time refers to the duration of time when the square pulse is above the baseline level. The "down" time refers to the duration of time when the square pulse has returned to and remains at the baseline level. Two-level RTS is defined as a Lorentzian spectrum in the frequency domain (Machlup, 1954). The Lorentzian spectrum gives the frequency above which the spectrum rolls-off as $f^{-2}$, conditioning the harmonic mean of both times, "up" and "down". When the RTS amplitude is sufficiently higher than the background noise, the Lorentzian spectrum can be used to estimate both time constants (Kolhatkar et al., 2003). A theoretical current PSD can also be derived from a 2-level signal as follows (Machlup, 1954):

$$1/\tau_{eff} = 1/\tau_{UP} + 1/\tau_{DOWN} \tag{2}$$

$$S_I(\omega) = 4(\delta I^2)\,(\tau_{eff}/(\tau_{UP}+\tau_{DOWN}) \cdot \tau_{eff}/(1+\omega^2\tau^2_{eff})) \tag{3}$$

where $\omega = 2\pi f$ is the angular frequency and $\mathcal{I}$ is the amplitude of the current pulses.

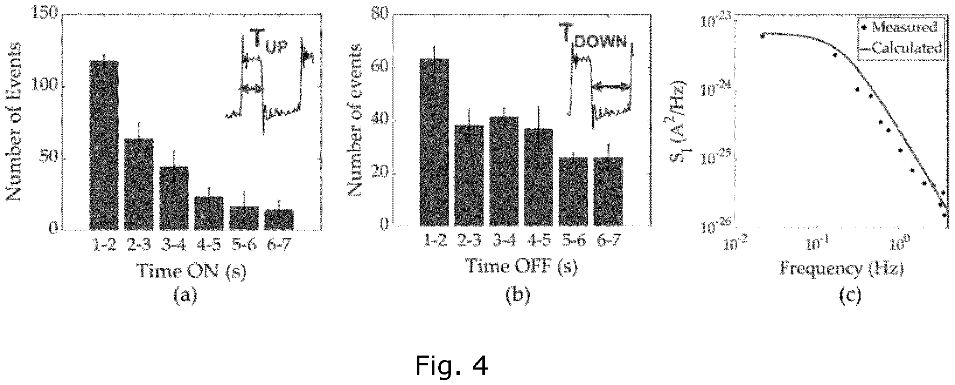

The second electrical pattern revealed a wide square pulse shape as depicted in Fig. 2(b) and Fig. 2(e). This pattern consists of square pulses that maintain their amplitude between two different and well-defined states (inset of Fig. 4(a)). The square-like pulses were characterised in terms of the length of time they are at an "up" state, $\tau_{UP}$, versus a "down" state, $\tau_{DOWN}$. Since spikes with pulse widths in the order of < 1 s are typically associated with previously seen VGSC activity, this analysis focused on pulses lasting 1 s or more. The characterisation of RTS is shown in Fig. 4(a) and Fig. 4(b) with the time at an "up" state being typically 1-3 s, and the time at an "down" state around 1-5 s. Occasionally, these time intervals reached durations of 5 seconds or more. Fig. 4(c) illustrates the PSD for the RTS noise, which shows the expected Lorentzian spectrum. The Lorentzian spectrum was calculated using Equation (3) and is represented as a solid black line. Using the values $\tau_{UP}$ = 1.1 s and $\tau_{DOWN}$ = 2.5 s, extracted from Fig. 4(a) and (b), one can model the RTS data and extract the overall characteristic times. The experimental data is plotted in black dots illustrating a good match between the analytical model and the experimental data.

The RTS shape can be explained as an extracellular travelling 241 wave across the large area electrode.

Considering the electrode area and duration of each pulse, it is possible to calculate the distance travelled by the signal and hence the wave speed. When a wave enters the measurement electrode, the potential increases relative to the counter electrode, resulting in an induced displacement current through the double layer capacitance. This appears as an increase in current in the electrical measurements and once the wave has left the measurement electrode, the current decreases back to its original level (Rocha *et al.,* 2016a; Cabello *et al.,* 2019). The "up" states shown in the inset of Fig. 4(a), which go from the start of a pulse to when it returns to baseline level, typically range between 1 and 3 s. These timings were obtained when performing measurements on the 2 mm$^2$ electrodes, which have a diameter of 1.6 mm. Therefore, the resultant wave speeds were of the order of hundreds of micrometres per second. This is in close agreement with reported values for intercellular $Ca^{2+}$ waves (Kuga *et al.,* 2011).

Conclusions and future perspective

The present experiments have revealed, for the first time, the extracellular electrical activity produced by MDA-MB-231 and MCF-7 cells, which was recorded using highly sensitive MEAs comprising gold electrodes with an area of 2 mm$^2$. It was found that the strongly metastatic MDA-MB-231 cells are electrically active with spontaneous spiking, whilst the MCF-7 cells showed only very low-level electrical excitability. This is consistent with MDA-MB-231 cells being electrically excitable in line with their metastatic ability (Djamgoz, 2011; Djamgoz et al., 2014). The electrical behaviour of MDA-MB-231 cells reveal an asynchronous pattern identified primarily as the product of their VGSC activity, as demonstrated by the TTX experiments. A slower signal pattern was also occasionally observed with similar characteristics to that of RTS noise. The calculated wave speed for the RTS pattern was in agreement with previously reported values for intercellular Ca$^{2+}$ waves. Ion channels involved in cell migration would be expected to regulate intracellular Ca$^{2+}$ signalling, most likely coupling extracellular stimuli to cell migration. Therefore, this work demonstrated the possibility using non-invasive, highly sensitive transducer devices to examine and pinpoint the origin of the electrical behaviour of cancer cells.

It is envisaged that in the future, it will be possible to use the sensor platform to investigate the pharmacological properties and action of drugs on cancer cell ensembles in a clinical setting.

LIST OF REFERENCES

- Al-Mahmood, S. et al. (2018). Drug Delivery and Translational Research 8, 1483-1507.
- Arcangeli, A. et al. (2008). Current Medicinal Chemistry.
- Bray, F. et al. (2018). Global cancer statistics 2018: GLOBOCAN estimates of incidence and mortality worldwide for 36 cancers in 185 countries. CA: a cancer journal for clinicians 68, 394-424.
- Cabello, M. et al. (2019). Sensors (Basel, Switzerland) 19.
- Chioni, A.-M. et al. (2009). The International Journal of Biochemistry & Cell Biology 41, 1216-1227.
- Cuddapah, V.A., and Sontheimer, H. (2011). American Journal of Physiology-Cell Physiology 301, C541-C549.
- Djamgoz, M.B.A. (2011). "Bioelectricty of Cancer: Voltage-Gated Ion Channels and Direct-Current Electric Fields," in The Physiology of Bioelectricity in Development, Tissue Regeneration and Cancer. (London and New York: Taylor & Francis), 269-294.
- Djamgoz, M.B.A., et al. (2014). Philosophical Transactions of the Royal Society B: Biological Sciences 369, 20130092.
- Djamgoz, M.B.A. et al. (2019). Cancers 11.
- Fraser, S.P. et al. (2005). Clinical Cancer Research: An Official Journal of the American Association for Cancer Research 11, 5381-5389.
- Fraser, S.P., and Pardo, L.A. (2008). EMBO reports 9, 512-515.
- Grimes, J.A. et al. (1995). FEBS letters 369, 290-294.
- Hero, T. et al. (2019). Anticancer Res 39, 2821-2827.
- Kolhatkar, J.S. et al. (2003). Essderc 2003: Proceedings of the 33rd European Solid-State Device Research Conference, 549-552.
- Kuga, N. et al. (2011). Journal of Neuroscience 31, 2607-2614.
- Lastraioli, E., et al. (2015). Biochimica et Biophysica Acta-Biomembranes 1848, 2685-2702.
- Lee, A., and Djamgoz, M.B.A. (2018). Cancer Treatment Reviews 62, 110-122.
- Machlup, S. (1954). Journal of Applied Physics 25, 341-343.
- Medeiros, M.C.R. et al. (2016). Sensing and Bio-Sensing Research 10, 1-8.
- Neve, R.M. et al. (2006). Cancer Cell 10, 515-527.
- Perou, C.M. et al. (2000). Nature 406, 747-752.
- Prevarskaya, N. et al. (2010). Trends in Molecular Medicine 16, 107-121.
- Rizaner, N. et al. (2016). European biophysics journal: EBJ 45, 735-748.
- Rocha, P.R.F. et al. (2016a). Extracellular electrical recording of pH-triggered bursts in C6 glioma cell populations. Science Advances 2, e1600516.
- Rocha, P.R.F. et al. (2016b). Scientific Reports 6, 34843.
- Roger, S. et al. (2003). Biochimica et Biophysica Acta 1616, 107-111.
- Sever, R. et al. (2015). Cold Spring Harbor Perspectives in Medicine 5.
- Sorlie, T. et al. (2001). Proceedings of the National Academy of Sciences of the United States of America 98, 10869-10874.
- Spira, M.E., and Hai, A. (2013). Nat Nanotechnol 8, 83-94.

- Yildirim, S. et al. Cancer Letters 323, 58- 61.

**Claims**

1. A method for determining the metastatic and/or invasive potential of a malignant neoplasm, the method comprising recording electrical activity in a sample of cells isolated from the malignant neoplasm and comparing recorded electrical activity of the sample with electrical activity of one or more reference cell samples having a defined metastatic and/or invasive potential and subsequently assigning a score for the metastatic and/or invasive potential of the malignant neoplasm by ranking the recorded electrical activity relative to the electrical activity of the one or more reference samples, wherein the reference cell samples preferably are at least a cell sample from a highly metastasizing cancer and a cell sample from a non-metastatic or low-metastasizing cancer, preferably a cancer of the same tissue origin and histological origin,
   wherein said electrical activity is constituted by variations in the membrane potential relative to the resting potential measured in the sample of cells.

2. The method according to claim 1, comprising comparing the recorded electrical activity to electrical activities from each of a plurality of reference cell samples.

3. The method according to any one of the preceding claims, wherein the malignant neoplasm is a breast cancer, and wherein the reference cell samples preferably include or consist of MDA-MB-231 and MCF-7 cells.

4. The method according to any one of the preceding claims, wherein the electrical activity recorded comprises amplitude and/or duration/width and/or frequency and/or number and/or shape and/or pattern of spikes of electric current or membrane potential.

5. The method according to claim 4, wherein assignment of the score of metastatic and/or invasive potential is based on any one of

   a) the amplitude of spikes of electric current or membrane potential,
   b) the duration/width of spikes of electric current or membrane potential,
   c) the frequency of spikes of electric current or membrane potential,
   d) the number of spikes of electric current or membrane potential,
   e) the shape of spikes of electric current or membrane potential, any
   f) the pattern of spikes of electric current or membrane potential,

   or a combination of 2, 3, 4, 5 or 6 of a-f.

6. The method according to any one of the preceding claims, wherein the electrical activity is measured by recording electric currents and/or potentials with a multi-electrode array capable of measuring low-frequency electrical events in the sample, such as events with a frequency <1 kHz, and wherein the electrodes of the multi-electrode array preferably each have contact areas >1 mm$^2$, such as about 2 mm$^2$.

7. The method according to claim 6, wherein the electrodes are covered by or made from an inert material such as gold, platinum, and rhodium.

8. The method according to any one of claims 4-7, wherein the cells of the sample adhere to the electrode during recording, and wherein the cells of the sample preferably form a confluent layer across the electrodes during recording.

9. The method according to any one of the preceding claims, wherein multiple recordings are made, wherein some recordings are made in the presence of a VGSC (voltage gated sodium channel) inhibiting agent, such as tetrodotoxin (TTX).

10. The method according to claim 9, wherein the electrical activity is recorded under a plurality of concentrations of the VGSC inhibiting agent and/or wherein the electrical activity is recorded in a period after addition of the VGSC inhibiting agent and/or in a period after the concentration of the VGSC inhibiting agent has been reduced.

11. A method for classifying the therapeutic potential of an agent or a combination of agents for prevention and/or inhibition of metastasis and/or invasive growth by a malignant neoplasm in a patient, the method comprising determining the metastatic and/or invasive potential of the malignant neoplasm according to the method of any one of the preceding claims under conditions 1) in the absence of the agent or combination of agents and 2) where the agent or combination of agents is or has been added, wherein the agent or combination of agents is classified as having a therapeutic potential for treatment of the patient if it decreases the assigned score for metastatic and/or invasive potential in an *in vivo* acceptable concentration, wherein the method preferably is carried out under hypoxic conditions.

**Patentansprüche**

1. Verfahren zum Feststellen des metastatischen und/oder invasiven Potenzials einer bösartigen Neubildung, wobei das Verfahren das Aufzeichnen von elektrischer Aktivität in einer Probe von Zellen, die aus der bösartigen Neubildung isoliert wurden, und das Vergleichen aufgezeichneter elektrischer Aktivität der Probe mit elektrischer Aktivität einer oder mehrerer Referenzzellproben mit einem definierten metastatischen und/oder invasiven Potenzial und das anschließende Zuweisen eines Werts für das metastatische und/oder invasive Potenzial der bösartigen Neubildung durch Einstufen der aufgezeichneten elektrischen Aktivität im Verhältnis zu der elektrischen Aktivität der einen oder mehreren Referenzproben umfasst, wobei es sich bei den Referenzzellproben bevorzugt um mindestens eine Zellprobe von einem stark metastasierenden Krebs und eine Zellprobe von einem nicht-metastasierten oder schwach metastasierenden Krebs, bevorzugt von einem Krebs aus demselben Gewebe oder mit demselben histologischen Ursprung, handelt,
wobei die elektrische Aktivität aus Variationen im Membranpotential relativ zum Ruhepotential, gemessen in der Probe von Zellen, besteht.

2. Verfahren nach Anspruch 1, umfassend das Vergleichen der aufgezeichneten elektrischen Aktivität mit elektrischen Aktivitäten von jeder aus einer Vielzahl von Referenzzellproben.

3. Verfahren nach einem der vorhergehenden Ansprüche, wobei es sich bei der bösartigen Neubildung um einen Brustkrebs handelt und wobei die Referenzzellproben bevorzugt MDA-MB-231- und MCF-7-Zellen umfassen oder daraus bestehen.

4. Verfahren nach einem der vorhergehenden Ansprüche, wobei die aufgezeichnete elektrische Aktivität eine Amplitude und/oder Dauer/Breite und/oder Frequenz und/oder Anzahl und/oder Form und/oder ein Muster von Spitzen von elektrischem Strom oder eines Membranpotentials umfasst.

5. Verfahren nach Anspruch 4, wobei die Zuweisung des Werts für metastatisches und/oder invasives Potenzial auf einem der Folgendem basiert:

   a) der Amplitude von Spitzen von elektrischem Strom oder eines Membranpotentials,
   b) der Dauer/Breite von Spitzen von elektrischem Strom oder eines Membranpotentials,
   c) der Frequenz von Spitzen von elektrischem Strom oder eines Membranpotentials,
   d) der Anzahl von Spitzen von elektrischem Strom oder eines Membranpotentials,
   e) der Form von Spitzen von elektrischem Strom oder eines Membranpotentials,
   f) dem Muster von Spitzen von elektrischem Strom oder eines Membranpotentials,

   oder einer Kombination aus 2, 3, 4, 5 oder 6 von a bis f.

6. Verfahren nach einem der vorhergehenden Ansprüche, wobei die elektrische Aktivität durch Aufzeichnen elektrischer Ströme und/oder Potentiale mit einer Multi-Elektrodenanordnung gemessen wird, die in der Lage ist, niederfrequente elektrische Ereignisse in der Probe zu messen, wie etwa Ereignisse mit einer Frequenz < 1 kHz, und wobei die Elektroden der Multi-Elektrodenanordnung bevorzugt jeweils Kontaktflächen > 1 mm$^2$, beispielsweise etwa 2 mm$^2$, aufweisen.

7. Verfahren nach Anspruch 6, wobei die Elektroden mit einem inerten Material wie Gold, Platin und Rhodium beschichtet sind oder aus einem solchen bestehen.

8. Verfahren nach einem der Ansprüche 4 bis 7, wobei die Zellen der Probe während des Aufzeichnens an der Elektrode haften bleiben und wobei die Zellen der Probe während des Aufzeichnens bevorzugt eine konfluente Schicht über den Elektroden bilden.

9. Verfahren nach einem der vorhergehenden Ansprüche, wobei mehrere Aufzeichnungen durchgeführt werden, wobei einige Aufzeichnungen in Gegenwart eines VGSC(spannungsgesteuerter Natriumkanal)-hemmenden Mittels, wie beispielsweise Tetrodotoxin (TTX), durchgeführt werden.

10. Verfahren nach Anspruch 9, wobei die elektrische Aktivität bei einer Vielzahl von Konzentrationen des VGSC-hemmenden Mittels aufgezeichnet wird und/oder wobei die elektrische Aktivität in einem Zeitraum nach Zugabe des VGSC-hemmenden Mittels und/oder in einem Zeitraum nach Verringerung der Konzentration des VGSC-hemmenden Mittels aufgezeichnet wird.

11. Verfahren zum Klassifizieren des therapeutischen Potenzials eines Mittels oder einer Kombination von Mitteln zur Verhinderung und/oder Hemmung von Metastasierung und/oder invasivem Wachstum einer bösartigen Neubildung bei einem Patienten, wobei das Verfahren das Bestimmen des metastatischen und/oder invasiven Potenzials der bösartigen Neubildung nach dem Verfahren eines der vorhergehenden Ansprüche unter den Bedingungen 1) in Abwesenheit des Mittels oder der Kombination von Mitteln und 2) unter Zugabe des Mittels oder der Kombination von Mitteln umfasst, wobei das Mittel oder die Kombination von Mitteln als ein therapeutisches Potenzial zur Behandlung des Patienten aufweisend klassifiziert wird, wenn es den zugewiesenen Wert für metastatisches und/oder invasives Potenzial in einer *in vivo* akzeptablen Konzentration verringert, wobei das Verfahren bevorzugt unter hypoxischen Bedingungen durchgeführt wird.

## Revendications

1. Procédé de détermination du potentiel métastatique et/ou invasif d'un néoplasme malin, le procédé comprenant l'enregistrement de l'activité électrique dans un échantillon de cellules isolées du néoplasme malin et la comparaison de l'activité électrique enregistrée de l'échantillon avec l'activité électrique d'un ou de plusieurs échantillons cellulaires de référence ayant un potentiel métastatique et/ou invasif défini et ensuite l'assignation d'un score pour le potentiel métastatique et/ou invasif du néoplasme malin en notant l'activité électrique enregistrée par rapport à l'activité électrique de l'un ou de plusieurs échantillons de référence, les échantillons cellulaires de référence étant de préférence au moins un échantillon cellulaire d'un cancer à haute métastase et un échantillon cellulaire d'un cancer non métastatique ou à faible métastase, de préférence un cancer de même origine tissulaire et origine histologique, ladite activité électrique étant constituée par des variations du potentiel membranaire par rapport au potentiel au repos mesuré dans l'échantillon de cellules.

2. Procédé selon la revendication 1, comprenant la comparaison de l'activité électrique enregistrée aux activités électriques de chacun d'une pluralité d'échantillons cellulaires de référence.

3. Procédé selon l'une quelconque des revendications précédentes, dans lequel le néoplasme malin est un cancer du sein et les échantillons cellulaires de référence comprenant de préférence ou étant constitués de cellules MDA-MB-231et MCF-7.

4. Procédé selon l'une quelconque des revendications précédentes, dans lequel l'activité électrique enregistrée comprend l'amplitude et/ou la durée/largeur et/ou la fréquence et/ou le nombre et/ou la forme et/ou le profil de pics de courant électrique ou de potentiel membranaire.

5. Procédé selon la revendication 4, dans lequel l'assignation du score de potentiel métastatique et/ou invasif est basé sur l'un quelconque de

a) l'amplitude des pics de courant électrique ou de potentiel membranaire,
b) la durée/largeur des pics de courant électrique ou de potentiel membranaire,
c) la fréquence des pics de courant électrique ou de potentiel membranaire,
d) le nombre des pics de courant électrique ou de potentiel membranaire,
e) la forme des pics de courant électrique ou de potentiel membranaire, l'une quelconque
f) le profil des pics de courant électrique ou de potentiel membranaire

ou une combinaison de 2, 3, 4, 5 ou 6 de a - f.

6. Procédé selon l'une quelconque des revendications précédentes, dans lequel l'activité électrique est mesurée en enregistrant les courants électriques et/ou les potentiels avec un réseau multi-électrodes capable de mesurer des événements électriques de faible fréquence dans l'échantillon, tels que des événements avec une fréquence < 1 kHz et les électrodes du réseau multi-électrodes ayant chacune de préférence des surfaces de contact > 1 mm$^2$, tel qu'environ 2 mm$^2$.

7. Procédé selon la revendication 6, dans lequel les électrodes sont recouvertes par ou constituées d'un matériau inerte tel que l'or, le platine et le rhodium.

8. Procédé selon l'une quelconque des revendications 4 à 7, dans lequel les cellules de l'échantillon adhèrent à l'électrode durant l'enregistrement, et les cellules de l'échantillon formant de préférence une couche confluente à travers les électrodes durant l'enregistrement.

9. Procédé selon l'une quelconque des revendications précédentes, dans lequel des enregistrements multiples sont effectués, certains enregistrements étant effectués en présence d'un inhibiteur de VGSC (canal sodique à porte de tension), tel que la tétrodotoxine (TTX).

10. Procédé selon la revendication 9, dans lequel l'activité électrique est enregistrée sous une pluralité de de concentrations de l'agent inhibiteur de VGSC et/ou l'activité électrique étant enregistrée dans une période après l'addition de l'agent inhibiteur de VGSC et/ou dans une période après que la concentration de l'agent inhibiteur de VGSC a été réduite.

11. Procédé de classification du potentiel thérapeutique d'un agent ou d'une combinaison d'agents pour la prévention et/ou l'inhibition de la métastase et/ou de la croissance invasive par un néoplasme malin chez un patient, le procédé comprenant la détermination du potentiel métastatique et/ou invasif du néoplasme malin selon le procédé de l'une quelconque des revendications précédentes dans les conditions 1) en absence de l'agent ou de la combinaison d'agents et 2) où l'agent ou la combinaison d'agents est ou a été ajouté, l'agent ou la combinaison d'agents étant classifié comme ayant un potentiel thérapeutique pour le traitement du patient s'il diminue le score assigné pour le potentiel métastatique et/ou invasif dans une concentration acceptable *in vivo,* le procédé étant de préférence réalisé sous des conditions hypoxiques.

Fig. 1

Fig. 2

(a)                Fig. 3                (b)

Fig. 4

EP 4 118 430 B1

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- EP 19205075

- WO 2012049440 A

## Non-patent literature cited in the description

- **AL-MAHMOOD, S. et al.** *Drug Delivery and Translational Research,* 2018, vol. 8, 1483-1507
- **ARCANGELI, A. et al.** *Current Medicinal Chemistry,* 2008
- **BRAY, F. et al.** Global cancer statistics 2018: GLOBOCAN estimates of incidence and mortality worldwide for 36 cancers in 185 countries. *CA: a cancer journal for clinicians,* 2018, vol. 68, 394-424
- **CABELLO, M. et al.** *Sensors,* 2019, 19
- **CHIONI, A.-M. et al.** *The International Journal of Biochemistry & Cell Biology,* 2009, vol. 41, 1216-1227
- **CUDDAPAH, V.A. ; SONTHEIMER, H.** *American Journal of Physiology-Cell Physiology,* 2011, vol. 301, C541-C549
- Bioelectricty of Cancer: Voltage-Gated Ion Channels and Direct-Current Electric Fields. **DJAMGOZ, M.B.A.** The Physiology of Bioelectricity in Development, Tissue Regeneration and Cancer. Taylor & Francis, 2011, 269-294
- **DJAMGOZ, M.B.A. et al.** *Philosophical Transactions of the Royal Society B: Biological Sciences,* 2014, vol. 369, 20130092
- **DJAMGOZ, M.B.A. et al.** *Cancers,* 2019, 11
- **FRASER, S.P. et al.** *Clinical Cancer Research: An Official Journal of the American Association for Cancer Research,* 2005, vol. 11, 5381-5389
- **FRASER, S.P. ; PARDO, L.A.** *EMBO reports,* 2008, vol. 9, 512-515
- **GRIMES, J.A. et al.** *FEBS letters,* 1995, vol. 369, 290-294
- **HERO, T. et al.** *Anticancer Res,* 2019, vol. 39, 2821-2827
- **KOLHATKAR, J.S. et al.** *Essderc 2003: Proceedings of the 33rd European Solid-State Device Research Conference,* 2003, 549-552

- **KUGA, N. et al.** *Journal of Neuroscience,* 2011, vol. 31, 2607-2614
- **LASTRAIOLI, E. et al.** *Biochimica et Biophysica Acta-Biomembranes,* 2015, vol. 1848, 2685-2702
- **LEE, A. ; DJAMGOZ, M.B.A.** *Cancer Treatment Reviews,* 2018, vol. 62, 110-122
- **MACHLUP, S.** *Journal of Applied Physics,* 1954, vol. 25, 341-343
- **MEDEIROS, M.C.R. et al.** *Sensing and Bio-Sensing Research,* 2016, vol. 10, 1-8
- **NEVE, R.M. et al.** *Cancer Cell,* 2006, vol. 10, 515-527
- **PEROU, C.M. et al.** *Nature,* 2000, vol. 406, 747-752
- **PREVARSKAYA, N. et al.** *Trends in Molecular Medicine,* 2010, vol. 16, 107-121
- **RIZANER, N. et al.** *European biophysics journal: EBJ,* 2016, vol. 45, 735-748
- **ROCHA, P.R.F. et al.** Extracellular electrical recording of pH-triggered bursts in C6 glioma cell populations. *Science Advances,* 2016, vol. 2, e1600516
- **ROCHA, P.R.F. et al.** *Scientific Reports,* 2016, vol. 6, 34843
- **ROGER, S. et al.** *Biochimica et Biophysica Acta,* 2003, vol. 1616, 107-111
- **SEVER, R. et al.** *Cold Spring Harbor Perspectives in Medicine,* 2015, 5
- **SORLIE, T. et al.** *Proceedings of the National Academy of Sciences of the United States of America,* 2001, vol. 98, 10869-10874
- **SPIRA, M.E. ; HAI, A.** *Nat Nanotechnol,* 2013, vol. 8, 83-94
- **YILDIRIM, S. et al.** *Cancer Letters,* vol. 323, 58-61